(19) Europäisches Patentamt
European Patent Office
Office européen des brevets

(11) **EP 4 125 000 A1**

(12) **EUROPEAN PATENT APPLICATION**

(43) Date of publication:
**01.02.2023 Bulletin 2023/05**

(21) Application number: **21196668.4**

(22) Date of filing: **14.09.2021**

(51) International Patent Classification (IPC):
**G06N 3/04** (2006.01)     **G06N 3/08** (2006.01)
**G16H 30/40** (2018.01)

(52) Cooperative Patent Classification (CPC):
**G06N 3/0454; G06N 3/08; G16H 30/40**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.07.2021 IN 202141034243**

(71) Applicant: **Koninklijke Philips N.V.**
**5656 AG Eindhoven (NL)**

(72) Inventors:
• **RUBIN, Jonathan**
**5656 AE Eindhoven (NL)**

• **CHEN, Alvin**
**5656 AE Eindhoven (NL)**
• **ERKAMP, Ramon Quido**
**5656 AE Eindhoven (NL)**
• **SRINIVASA NAIDU, Raghavendra**
**5656 AE Eindhoven (NL)**
• **ODUNGATTU THODIYIL, Anumod**
**5656 AE Eindhoven (NL)**

(74) Representative: **Philips Intellectual Property & Standards**
**High Tech Campus 52**
**5656 AG Eindhoven (NL)**

(54) **GENERATING LOCATION DATA**

(57)     In an embodiment, a computer-implemented method (100) is described. The method (100) comprises receiving (102) input data. The method 100 further comprises generating (104) location data indicative of a location of any detected at least one feature of interest in the received input data. The location data is generated using a first machine learning, ML, model configured to detect whether or not there is at least one feature of interest in the received input data. The first ML model is trained based on a learning process implemented by a second ML model configured to detect whether or not there is at least one feature of interest in the received input data. The first ML model and the second ML model are each configured to use an attention mechanism to generate: at least one attention map from at least one layer of the first ML model; and a plurality of attention maps from a plurality of layers of the second ML model. The first ML model comprises fewer layers than the second ML model. At least one attention map generated by the second ML model is used to train the first ML model.

**Fig. 3**

**Description**

FIELD OF THE INVENTION

The invention relates to a method, a non-transitory machine-readable medium and apparatus for generating location data.

BACKGROUND OF THE INVENTION

In certain imaging techniques, object detection may be used to detect and classify objects in imaging data. In medical imaging applications such as ultrasound imaging, object detection may be used to assist an operator such as a clinician when carrying out a procedure. Machine learning models may be deployed to provide such object detection. Although machine learning models could provide useful functionality, such models may have a large size, which may lead to difficulties in using such models in certain scenarios. For example, simultaneous real-time imaging and object detection may not be possible due to the size of the models and memory/processing constraints, especially when imaging complex scenarios such as in certain medical images.

Certain types of applications that may use machine learning models such as used in audio signal processing, natural language processing (NLP), machine translation services and other types of signal processing may experience difficulties if deployed on equipment with memory/processing constraints.

SUMMARY OF THE INVENTION

Aspects or embodiments described herein may relate to improving the deployment and use of machine learning models in certain settings. Aspects or embodiments described herein may obviate one or more problems associated with using and/or training certain machine learning models in certain settings such as where there may be a memory and/or processing constraint. Certain technical benefits of certain aspects or embodiments are described below.

In a first aspect, a method is described. The method is a computer-implemented method. The method comprises receiving input data. The method further comprises generating location data indicative of a location of any detected at least one feature of interest in the received input data. The location data is generated using a first machine learning, ML, model configured to detect whether or not there is at least one feature of interest in the received input data. The first ML model is trained based on a learning process implemented by a second ML model configured to detect whether or not there is at least one feature of interest in the received input data. The first ML model is configured to use an attention mechanism to generate at least one attention map from at least one layer of the first ML model. The second ML model is configured to use an attention mechanism to generate a plurality of attention maps from a plurality of layers of the second ML model. The first ML model comprises fewer layers than the second ML model. At least one attention map generated by the second ML model is used to train the first ML model.

Some embodiments relating to the first aspect and other related aspects are described below.

In some embodiments, the first and second ML models are based on a detection transformer architecture. The at least one layer of the first and second ML models may comprise a transformer layer.

In some embodiments, the detection transformer architecture comprises a backbone neural network configured to down-sample the input data to produce a tensor of activations for processing by the at least one transformer layer of the first and second ML models. The at least one transformer layer of the first and second ML models may be based on an encoder-decoder transformer architecture for predicting the location of the at least one feature of interest and/or outputting data representative of the predicted location of the at least one feature of interest.

In some embodiments, the method comprises comparing attention maps generated by the first and second ML models to determine whether or not the first ML model meets a similarity metric indicative of similarity between the compared attention maps. In response to determining that the first ML model does not meet the similarity metric, the method may further comprise updating the at least one layer of first ML model using the at least one attention map generated by the second ML model.

In some embodiments, the similarity metric is based on a Kullback-Leibler, KL, divergence score.

In some embodiments, the KL divergence score comprises a first component and a second component. The first component may be configured to apply knowledge distillation to the at least one attention map generated by the at least one layer of the first and second ML models by attempting to match the attention maps generated by the first and second ML models. The second component may be configured to apply knowledge distillation to class label predictions.

In some embodiments, the first ML model is updated by modifying a loss function used to train the first ML model based on the similarity metric. The loss function may be further based on ground-truth target data. The method may comprise using a hyper-parameter to control mixing between loss based on the similarity metric and loss based on the ground-truth target labels when training the first and second ML models.

In some embodiments, the at least one attention map generated by the second ML model used to train the first ML model is distilled from the plurality of attention maps generated by the second ML model.

In some embodiments, the method comprises generating an attention map representative of the generated location data. The attention map may be generated by using the first ML model. In some cases, the attention map may be generated by using the second ML model.

In some embodiments, the attention map is generated by at least one encoder of the at least one layer. In some embodiments, the attention map is generated by at least one decoder of the at least one layer. In some embodiments, the attention map is generated based on a combination of the at least one encoder and decoder of the at least one layer.

In some embodiments, the method comprises causing a display to show the generated attention map.

In some embodiments, the received input data comprises three-dimensional data and/or temporal data used by the second ML model. The method may further comprise implementing a convolution procedure to reduce the received input data to a dimensional format for use by the first ML model.

In some embodiments, the method comprises receiving an indication to use the second ML model instead of the first ML model to generate the location data from the received input data. In response to receiving the indication, the method may comprise generating the location data using the second ML model.

In a second aspect, a non-transitory machine-readable medium is described. The non-transitory machine-readable medium stores instructions executable by at least one processor. The instructions are configured to cause the at least one processor to receive input data. The instructions are further configured to cause the at least one processor to generate location data indicative of a location of any detected at least one feature of interest in the received input data. The location data is generated using a first machine learning, ML, model configured to detect whether or not there is at least one feature of interest in the received input data. The first ML model is trained based on a learning process implemented by a second ML model configured to detect whether or not there is at least one feature of interest in the received input data. The first ML model is configured to use an attention mechanism to generate at least one attention map from at least one layer of the first ML model. The second ML model is configured to use an attention mechanism to generate a plurality of attention maps from a plurality of layers of the second ML model. The first ML model comprises fewer layers than the second ML model. At least one attention map generated by the second ML model is used to train the first ML model.

In a third aspect, apparatus is described. The apparatus comprises at least one processor communicatively coupled to an interface. The interface is configured to receive input data. The apparatus further comprises a non-transitory machine-readable medium. The non-transitory machine-readable medium stores instructions readable and executable by the at least one processor. The instructions are configured to cause the at least one processor to generate location data indicative of a location of any detected at least one feature of interest in the received input data. The location data is generated using a first machine learning, ML, model configured to detect whether or not there is at least one feature of interest in the received input data. The first ML model is trained based on a learning process implemented by a second ML model configured to detect whether or not there is at least one feature of interest in the received input data. The first ML model is configured to use an attention mechanism to generate at least one attention map from at least one layer of the first ML model. The second ML model is configured to use an attention mechanism to generate a plurality of attention maps from a plurality of layers of the second ML model. The first ML model comprises fewer layers than the second ML model. At least one attention map generated by the second ML model is used to train the first ML model.

Certain aspects or embodiments may provide at least one of the following technical benefits, as described in more detail below. (1) Compression of models (e.g., machine learning-based object detection models) according to certain embodiments e.g., for improved distribution and use of such models. (2) Improved precision using the smaller/faster/less expensive models trained in accordance with certain embodiments. (3) Utilizing performance gains (e.g., average precision scores) of large/complex models in smaller/faster/less expensive lightweight models. (4) Leveraging information from higher-dimension (e.g., image or video-based) detection models for use in lower-dimension detection models. (5) Reducing computational complexity so that the detections can be made in real time on lightweight processors, such as used in medical apparatus such as ultrasound apparatus. (6) Leveraging information generated anyway (e.g., 'by-product' information) by larger models to improve the performance of smaller models. (7) Certain output such as 'location information' may be displayed to support human interpretation of the model predictions. (8) Allowing an automatic or manual selection of different model types (e.g., large or small) depending on the use case. (9) Certain models may support a clinician during a medical imaging procedure, which may improve patient outcome and/or experience. Any combination of the above technical benefits (and further technical benefits) may be provided by certain embodiments described herein.

These and other aspects of the invention will be apparent from and elucidated with reference to the embodiment(s) described hereinafter.

BRIEF DESCRIPTION OF DRAWINGS

Exemplary embodiments of the invention will now be described, by way of example only, with reference to the following drawings, in which:

Fig. 1 refers to a method of generating location data according to an embodiment;
Fig. 2 is a schematic drawing of a system for generating and visualizing location data according to an embodiment;
Fig. 3 is a schematic illustration of an architecture for at least partially implementing certain embodiments described herein;
Fig. 4 depicts an example use case of generating location data according to an embodiment;
Fig. 5 depicts an example use case of generating location data and classification according to an embodiment;
Figs. 6 and 7 are graphs of data from needle detection experiments implementing certain embodiments described herein;
Figs. 8 and 9 highlight the speed and average precision results of the experiments;
Fig. 10 refers to a method of determining a similarity metric according to an embodiment;
Fig. 11 refers to a method of using a hyper-parameter according to an embodiment;
Fig. 12 refers to a method of generating an attention map according to an embodiment;
Fig. 13 refers to a method of showing an attention map according to an embodiment;
Fig. 14 refers to a method of implementing a convolution procedure according to an embodiment;
Fig. 15 refers to a method of selecting a machine learning model according to an embodiment;
Fig. 16 is a schematic drawing of a machine-readable medium for generating location data according to an embodiment; and
Fig. 17 is a schematic drawing of apparatus for generating location data according to an embodiment.

DETAILED DESCRIPTION OF THE EMBODIMENTS

As noted above, there may be issues with using and/or training certain machine learning models in certain settings such as where there may be a memory and/or processing constraint. Such issues may be applicable to imaging scenarios (e.g., including medical imaging) and other scenarios (e.g., audio signal processing, natural language processing (NLP), machine translation services and other types of signal processing). The following description refers to imaging scenarios but the concepts described herein may also apply to other scenarios.

Artificial intelligence (AI)-based object detection may be used in certain medical imaging solutions to provide object detection functionality that may be useful for an operator such as a clinician. Examples of AI-based object detectors include Faster Region based Convolutional Neural Networks (R-CNN), Single Shot Detector (SSD) and You Only Look Once (YOLO). Video-based object detectors may be used for imaging modalities that have a temporal component, for example ultrasound imaging.

One class of object detector is known as a DEtection TRansformer (i.e., 'DETR'). The DETR architecture is described in Carion et al., "End-to-end object detection with transformers", European Conference on Computer Vision, pages 213-229, Springer, 2020, the entire content of which is incorporated herein by reference. Detection transformers may not require anchor boxes or post-processing steps such as non-maximum suppression. Instead, they may rely on self-attention, along with bipartite matching and direct set prediction, to improve learning capacity and simplify the final bounding box calculation. An example by-product of such detection transformers is an 'attention map' or another type of 'location information' providing a representation of object locations and/or appearances. Other types of such 'location information' include other types of maps (e.g., a self-attention map, saliency map, context map, feature map, heat map, loss map, etc.) and encoding information (e.g., location encoding information that may be indicative of a part of data that is representative of object locations, appearances, etc., or any other contextual information about the data). An attention map (and indeed certain other types of maps/encoding information) may be regarded as providing a high-content representation of object locations and appearances.

One possible shortcoming of large transformer models such as implemented by the DETR architecture is that they may require extensive amounts of computation, making them expensive to train and use, particularly when inputs into the network are large. Detection transformers may be particularly susceptible to the above computational issues due to the large input sizes required by these networks. Detection transformers may not be amenable to real-time usage, especially on resource-limited hardware.

This disclosure proposes at least one solution to provide e.g., object detection functionality in a light-weight (e.g., compressed) model while also taking advantage of certain features of certain architectures (e.g., improved precision, etc.). The at least one solution may be applicable to imaging (e.g., medical imaging) and various types of applications in signal processing. Embodiments described below primarily refer to imaging applications but such embodiments could extend to other applications.

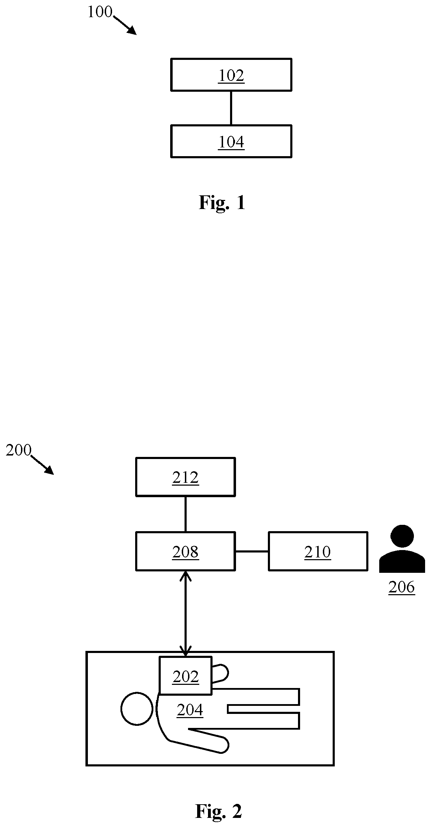

Fig. 1 refers to a method 100 of generating location data according to an embodiment. The method 100 is a computer-implemented method, which may be implemented on a computing device such as described below. For example, the method 100 may be implemented by a user computer (e.g., associated with use by a clinician such as a radiologist), a server, a cloud-based service and/or any other computing device whether in the locality of a radiographic imaging apparatus for acquiring data (e.g., imaging data) or remote from this locality.

The method 100 comprises, at block 102, receiving input data.

The received input data may refer to imaging data produced by an imaging apparatus (such as a radiographic imaging apparatus) for processing as described below. The input data may take various forms. For example, in the case of imaging data or video data, the input data may have one, two or three spatial dimensions and/or one temporal dimension. Pre-processing may be applied to change the dimensionality of the input data according to the implementation, as described in more detail below.

A first machine learning, ML, model is configured to detect whether or not there is at least one feature of interest in the received input data. For example, the received input may or may not have at least one feature of interest (e.g., an anatomical feature or structure, medical instrument such as a needle, etc.). The first ML model may be trained based in the received input data (and may be based on any other training data previously received such as previously-received input data, historical data and/or expert input) to detect whether or not the input data comprises at least one feature of interest.

The method 100 further comprises generating, at block 104, location data indicative of a location of any detected at least one feature of interest in the received input data. The location data is generated using the first ML model, which is configured to detect whether or not there is at least one feature of interest in the received input data. Thus, the first ML model may determine the location data of any detected at least one feature of interest.

Further features of the method 100 are described below with further explanations highlighted as optional features of the method 100.

In some cases, the location data may comprise a map such as an attention map as described in more detail below.

Any reference herein to an 'attention map' may also refer to 'location information' such as the various types of 'maps' and 'encoding information' described above. Thus, any reference to the term 'attention map' may, where appropriate, be replaced by the term 'location information'.

In some cases, the location data may be used for depicting or otherwise highlighting the location of the at least one feature of interest (if there is at least one feature of interest) in the input data.

The first ML model is trained based on a learning process implemented by a second ML model configured to detect whether or not there is at least one feature of interest in the received input data. The second ML model may be referred to as a 'teacher' model (or 'teacher network') and the first ML model may be referred to as a 'student' model (or 'student network'). Thus, the second ML model may teach or otherwise cause the first ML model to learn based on the result of the second ML model learning from the input data.

The first ML model and the second ML model are each configured to use an attention mechanism to generate at least one attention map. The first ML model uses the attention mechanism to generate at least one attention map from at least one layer of the first ML model. The second ML model uses the attention mechanism to generate a plurality of attention maps from a plurality of layers of the second ML model. Such attention maps may be regarded as a 'by-product' of the learning process.

The first ML model comprises fewer layers than the second ML model. For example, the first ML model (e.g., the part of the first ML model that performs object detection) may have a single layer whereas the second ML model may have more than one layer. Other combinations are possible. For example, the first ML model may comprise 2, 4 or 6 layers (e.g., as described below) and the second ML model may comprise 12 layers.

At least one attention map generated by the second ML model is used to train the first ML model.

An explanation of the deployment and use of embodiments that implement method 100 is provided below.

Since the second ML model may be a bigger model than the first ML model (e.g., since the second ML model has more layers), the second ML model may provide accurate/reliable detection of whether or not there are any features of interest in the input data (as well as indicating the location of such features, if any exist). However, the size of the second ML model may be prohibitive to deployment for real-time detection, especially if the deployed device does not have a suitable hardware for implementing the second ML model. This may be relevant for certain imaging apparatus such as ultrasound imaging apparatus.

The method 100 may therefore leverage knowledge distillation from the second ML model in order to improve training and/or use of the first ML model. Knowledge distillation may be regarded as a type of model compression technique where a small student network attempts to extract encoded knowledge from single or multiple large teacher model(s).

The use of knowledge distillation for tasks such as image or video object detection may be difficult due to challenges in designing a distillation technique that works well with certain object detector architectures based on anchor boxes and/or anchor grids. Distilling based on common bounding box or anchor box losses may require introducing additional elements into the object detector architectures, which may add further complexity in the design, optimization, and deployment of these models.

However, certain embodiments described herein (including method 100) may provide a machine learning model compression and/or distribution method to create fast and efficient image and video object detectors. Certain embodiments may allow small, fast and/or lightweight detector models to achieve up to the performance of more complex models (which may be large, slow and/or expensive) while still being able to run in real-time on resource-limited hardware. Certain embodiments may allow selection of the optimum type of model (e.g., small/lightweight or complex/expensive) for the use case.

The location information (e.g., attention maps, etc.) generated by certain object detection models such as transformer models can be re-purposed and used for knowledge distillation. As described herein, attention maps generated by the second ML model are used for what-is-termed 'attention distillation'. The technique of 'distillation' may also be applied to the other types of location information (e.g., other maps or encoding information) described herein. Embodiments described herein may support both image-to-image (2D-to-2D or 3D-to-2D) model compression and video-to-image (3D-to-2D) model compression.

Accordingly, certain embodiments described herein may provide at least one of the following features and/or functionality: (1) Compression of models (e.g., machine learning-based object detection models) according to certain embodiments e.g., for improved distribution and use of such models. (2) Improved precision using the smaller/faster/less expensive models trained in accordance with certain embodiments. (3) Utilizing performance gains (e.g., average precision scores) of large/complex models in smaller/faster/less expensive lightweight models (e.g., by efficiently distilling object detection knowledge into smaller and faster lightweight models). (4) Leveraging information from higher-dimension (e.g., image or video-based) detection models for use in lower-dimension detection models (e.g., by distilling 3D information and/or temporal information into 2D detectors). (5) Reducing computational complexity so that the detections can be made in real time on lightweight processors, such as used in medical apparatus such as ultrasound apparatus. (6) Leveraging information generated anyway (e.g., 'by-product' information) by larger models to improve the performance of smaller models. (7) Certain output such as 'location information' (e.g., the 'attention maps' or other types of 'location information') may be displayed to support human interpretation of the model predictions. (8) Allowing an automatic or manual selection of different model types (e.g., large or small) depending on the use case. (9) Certain models may support a clinician during a medical imaging procedure, which may improve patient outcome and/or experience. Any combination of the above technical benefits (and further technical benefits) may be provided by certain embodiments described herein. Any combination of the above features and/or functionality may be provided by certain embodiments described herein.

Other embodiments are described below. The following is a description of a deployed system that may implement the method 100 and/or certain other embodiments described herein.

Fig. 2 shows an example system 200 for implementing certain embodiments described herein (e.g., the method 100 and certain other embodiments). The system 200 comprises a radiographic imaging apparatus 202 such as an ultrasound imaging apparatus (or other apparatus such as a magnetic resonance imaging (MRI) scanner, computed tomography (CT) scanner, etc.) for imaging a subject 204 such as a patient. In use of the system 200, an operator 206 such as a clinician may perform a procedure such as an imaging procedure (e.g., in conjunction with a medical procedure such as needle biopsy in some cases) using the radiographic imaging apparatus 202. Embodiments described herein may provide certain object detection functionality, which may be helpful for the operator 206 when performing the procedure, which may also provide a beneficial outcome for the subject 204 (e.g., less time spent under the procedure and/or improved health outcome).

The radiographic imaging apparatus 202 is communicatively coupled to a controller 208 (which is an example of a 'computing device' as referred to in certain embodiments) for sending/receiving data (such as control data for controlling/monitoring the operation of the radiographic imaging apparatus 202 and/or imaging data acquired by the radiographic imaging apparatus 202) to/from the radiographic imaging apparatus 202. The controller 208 is communicatively coupled to a user interface such as a display 210 for displaying imaging data and/or other information associated with use of the system 200. Although the radiographic imaging apparatus 202 and the controller 208 are depicted as separate devices in Fig. 2, in some cases, they could be the same device (e.g., the radiographic imaging apparatus 202 may comprise the controller 208 and/or provide certain functionality of the controller 208).

In some cases, as shown by Fig. 2, the controller 208 may be communicatively coupled to an optional service provider 212 (e.g., a manufacturer of the radiographic imaging apparatus 202 or other entity that may control/monitor/perform data processing in relation to the radiographic imaging apparatus 202 and/or the controller 208). The service provider 212 may be a server or cloud-based service to which the controller 208 may be connected to exchange various data such as imaging data acquired by the radiographic imaging apparatus 202 and/or control data associated with controlling the radiographic imaging apparatus 202. In some cases, the service provider 212 may provide a data processing service (e.g., to at least partially implement certain embodiments described herein). In some cases, the controller 208 may perform data processing (e.g., to at least partially implement certain embodiments described herein). In some cases, the controller 208 and the service provider 212 may exchange data and/or collectively perform/facilitate data processing in order to implement certain embodiments described herein. In some cases, the controller 208 may receive updates (e.g., software updates, etc.) from the service provider 212. Such updates may include information relating to new and/or updated models (e.g., trained machine learning models, information about trained machine learning models (as referred to in certain embodiments described herein) and/or parameters such as neural network weights for the controller 208 to implement such trained machine learning models). Thus, in one scenario, the service provider 212 may assist with training a machine learning model (e.g., such as according to certain embodiments described herein) and then send the trained machine learning model (or location information such as the 'attention map' described herein in order to train the first ML model) to the controller 208 (e.g., to allow the controller 208 to at least partially implement a similar model as the second ML model). In another scenario, the controller 208 may be pre-loaded with the trained machine learning model. In another scenario, the controller 208 may not implement the machine learning model; instead the service provider 212 may implement the trained machine learning model (e.g., based on imaging data sent by the controller 208 to the service provider 212). Thus, the controller 208 and/or the service provider 212 may implement the 'computing device' referred to in various embodiments described herein (e.g., the method 100 and other embodiments described herein).

The controller 208 and the service provider 212 (if present) may each comprise processing circuitry (such as at least one processor, not shown) configured to perform data processing for implementing certain embodiments described herein. The controller 208 and/or the service provider 212 may comprise or have access to a memory (e.g., a non-transitory machine-readable medium) storing instructions which, when executed by the processing circuitry, causes the processing circuitry to implement certain embodiments described herein.

In some cases, the controller 208 may be implemented by a user computer. In some cases, the controller 208 and/or the service provider 212 may be implemented by a server or cloud-based computing service. In some cases, a memory (such as the non-transitory machine-readable medium described above and/or another memory such as another non-transitory machine-readable medium or a transitory machine-readable medium) may store information relating to the machine learning model (e.g., the machine learning model itself and/or output from such a model) and/or other data such as imaging data associated with the radiographic imaging apparatus 202.

Certain principles of knowledge distillation as implemented by certain embodiments is described below.

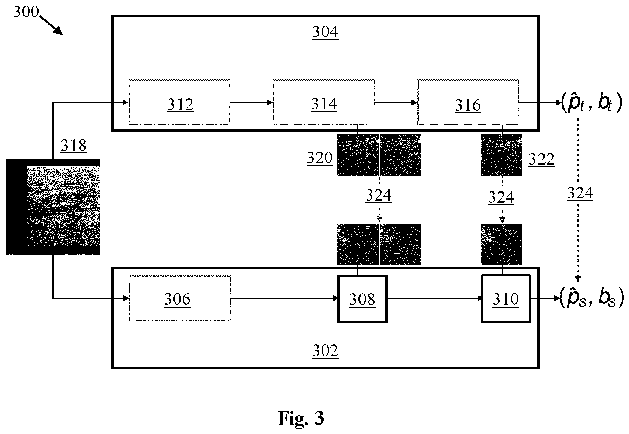

Fig. 3 is a schematic illustration of an architecture 300 for at least partially implementing certain embodiments described herein.

The architecture comprises a student network 302 (i.e., comprising the 'first ML model') and teacher network 304 (i.e., comprising the 'second ML model'). The student network 302 comprises a backbone 306 (e.g., comprising a convolutional neural network) for performing convolutional operations (e.g., image down-sampling, learning about input data, creating a smaller matrix/feature map, etc.). The student network 302 further comprises an encoder 308 and a decoder 310. The output from the backbone 306 (e.g., 'input data' such as in the form of an unrolled vector) is fed into the encoder 308, which is configured to capture information about the representation in the input data by using an attention mechanism (or another mechanism associated with providing the 'location information' as described herein). This information capture can be performed by examining every pixel (in the case of imaging data) and determining which pixels to pay most (or least) attention to. Thus, by 'paying attention' to (or otherwise examining) certain parts of the input data, the information capture mechanism may learn something about the input data. The decoder 310 takes the output from the encoder 308 and considers the pixels of interest to determine the output (i.e., predictions of the student's network 302, $\hat{p}_s$, and how these compare with the ground-truth target data, $b_s$). The teacher network 304 may have substantially the same architecture as the student network 302. Thus, the teacher network 304 comprises a backbone 312, encoder 314 and decoder 316 that respectively corresponds to the backbone 306, encoder 308 and decoder 310 of the student network 302. The output of the teacher network 304 is similar and is labelled ($\hat{p}_t$ and $b_t$, for the output predictions and ground truth target, respectively). Depending on the dimensionality of the input data, and requirements on memory and speed, the backbones 306, 312 may differ to accommodate processing of the data so that it is in a suitable for processing by the latter parts of the networks 302, 304.

The same images/video 318 (i.e., 'input data') are fed into both the student and teacher networks 302, 304. Thus, both networks 302, 304 attempt to train themselves based on the same data. However, the teacher network 304 comprises more layers and therefore has a higher learning capacity, which may lead to improved performance for the teacher network 304 (e.g., better accuracy than the student network 302). On the other hand, the fewer layers of the student network 302 may facilitate deployment on apparatus with certain hardware constraints.

Each of the encoders 308, 314 comprises at least one layer, each of which produces an attention map 320. At least one attention map (e.g., the final attention map of the final layer of the encoder 314) output by the teacher network 304 may be used for attention distillation so that the corresponding encoder 308 of the student network 302 learns based on an attention map that is more likely to be optimized or more accurate than the attention map produced by the encoder 308. A similar principle applies to the attention maps 322 produced by the at least one layer of the decoders 310, 316. Accordingly, the student network 302 may provide more accurate predictions, $\hat{p}_s$, by leveraging the attention maps of the teacher network 304. Such attention maps may be considered to be 'by-products' of the learning process. Leveraging such maps (or indeed any other 'location information' generated by other architectures) may not involve producing any additional information when implementing the models (i.e., such maps may be a natural consequence of machine learning and may otherwise not be used in any other way other than the learning process). For example, such location information may be used for knowledge distillation.

In other words, the teacher network 304 may provide a large object detection model adapted to output at least one self-attention map. The student network 302 may provide a (relatively) smaller object detection model adapted to output a same-sized attention map. A learning procedure (e.g., attention distillation) that compares the dissimilarity of attention maps from the teacher network 304 and student network 302 may be used, which updates the student network 302 so as to reduce or minimize this dissimilarity.

As part of the training process, a loss function may be used to direct each of the first and second ML models to focus on learning about certain parts of the input data. The dissimilarity between teacher and student self-attention maps may be directly incorporated into the loss function. Optionally, this 'distillation loss' may be combined with the loss based on ground-truth targets such as the bounding box produced by certain object detectors.

Optionally, certain data generated by the architecture 300 may be used for visual display purposes. For example, in the case of the 'location information' comprising an attention map, the distilled attention map (i.e., such attention maps may be based on 'location data indicative of a location of the any detected at least one feature of interest in the received input data' as referred to in block 104 of the method) generated by the student network 302 (e.g., via the display 210 of Fig. 2) may be displayed. Thus, such distilled attention maps (produced by either the student network 302 or teacher network 304) may provide a visual output for an end-user such as the operator 206, e.g. to facilitate human interpretation of the AI-based predictions. In some cases, the visual attention maps may replace the bounding box predictions as the primary output of certain object detectors.

Optionally, the architecture 300 may facilitate selection/switching between the teacher and student networks 304, 302, depending on the accuracy vs. speed demands for any given scenario. This selection could be made by the user, or done automatically.

Optionally, the large teacher network 304 may be a two-dimensional (2D) (e.g., single-frame) model or a three-dimensional (3D) (multi-frame or temporal) model. The student network 302 may also comprise a 2D or 3D model, although providing the student network 302 as a 2D model may help with achieving faster operating speeds e.g., for real-time usage.

Optionally, the 3D-to-2D attention distillation scenario may be relevant for fast video detection (e.g., as might be used in ultrasound imaging). This may be useful for data intense scenarios such as distillation of temporal information from a large 3D model that processes video into a fast 2D model that processes single frames (that may not otherwise have access to temporal context).

Optionally, the 3D-to-3D attention distillation scenario may also be relevant for fast video detection (e.g., as might be used in ultrasound imaging). This may be useful for distillation of temporal information from a large 3D model that processes video into a much smaller 3D model that processes substantially fewer video frames or video frames of lower dimensionality.

The architecture 300 may, in some applications, provide for fast image and/or video object detection to the extent that this can be run in real-time.

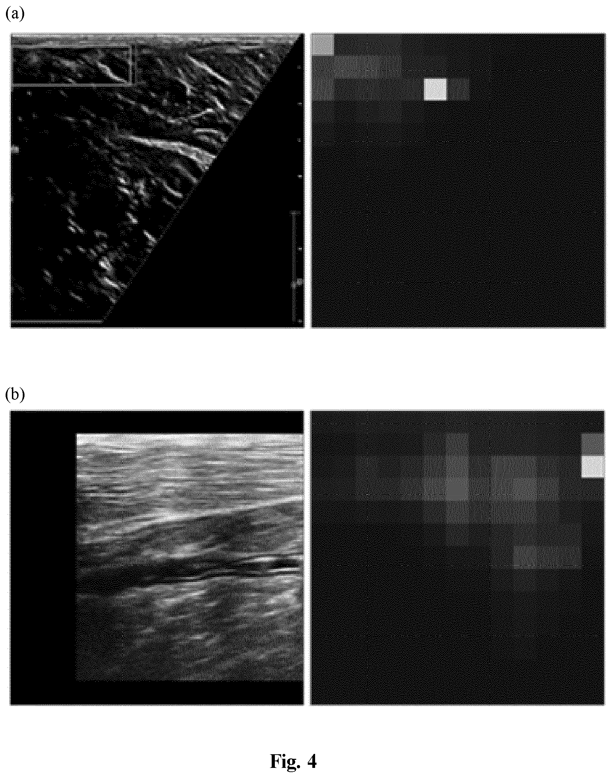

Fig. 4 depicts an example use case of generating location data (e.g., attention maps) according to an embodiment. Two use cases (a) and (b) are depicted in Fig. 4, each of which relate to needle detection in ultrasound imaging data. The images in Fig. 4 may represent challenging detection scenarios. In case (a), a needle is present in the top left corner of the image (left-hand side of Fig. 4(a)) and the corresponding attention map (which is depicted as a 'heat map' on the right-hand side of Fig. 4(a)) is generated according to certain embodiments described herein. The pixels depicted by the attention map depict the parts of the image to pay the highest 'attention' to (i.e., the 'higher intensity' pixels represent the pixels to pay the highest attention to). The two highest intensity pixels in each attention map depict the upper left and lower right corners of the bounding box visible in the image. In case (b), a small portion of a needle is present in the top right of the image. Again, the attention map depicts the position of the needle.

Needle detection is an example of a challenging use case of real-time ultrasound video detection. Clinically, false needle detections are an injury risk, and very fast speeds (e.g., screen refresh rates of e.g., >50 Hz) may be needed on lightweight processors in certain ultrasound systems. Experiments, described later, demonstrate notable gains in accuracy and/or speed compared to models developed without attention distillation. Thus, certain embodiments described herein may be used for improved needle detection during real-time imaging, as one example application of the embodiments.

The ability to reliably and instantaneously detect the presence and location of needles in noisy confounding ultrasound videos may open up a wide range of capabilities that may be useful for operators in various clinical settings.

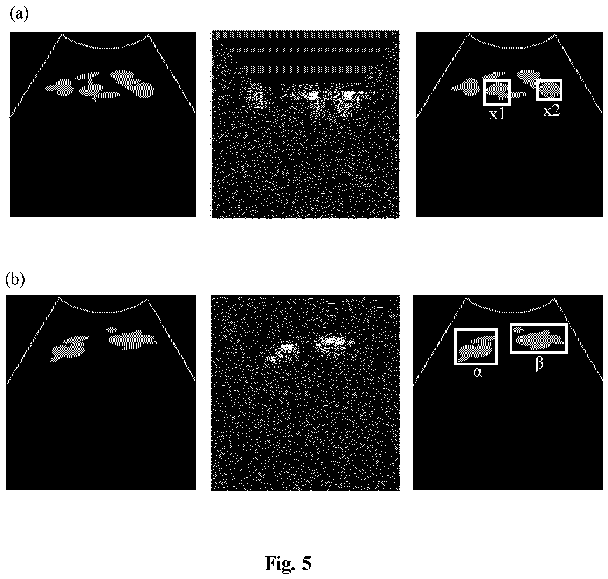

Fig. 5 depicts an example use case of generating location data (e.g., attention maps) and classification according to an embodiment. Two use cases (a) and (b) are depicted in Fig. 5, each of which relate to anatomy/pathology detection in ultrasound imaging data that is depicted in highly schematic form without any other structures of interest in the image. The image (left-hand side) in cases (a) and (b) schematically depict certain ultrasound pathologies. The central images are the corresponding attention maps for the respective schematic images used as input data, similar to Fig. 4. In addition, the right-hand side images are annotated with bounding boxes. Case (a) includes bounding boxes depicted the size (x1, x2) of certain features and case (b) includes bounding boxes with clinical annotations (i.e., pathology labelled '$\alpha$' and pathology labelled '$\beta$') that have been detected from classification of the input data. The labelled pathologies represent anatomical features and/or pathology classified by the detector.

As such, in some embodiments, the attention maps may provide a high-content and visually explainable representation of object locations and/or appearances. As such, they may increase the transparency of AI-based prediction by helping end-users to understand the salient features used by the model for prediction.

In some cases, the attention maps could be the primary output (e.g., replacing the bounding box detection altogether). This may reduce regulatory burden since software that provides attention maps may result in lower medical device regulatory classification than a software that outputs bounding boxes, which may be considered diagnostic. Processing/memory resource usage may be reduced if attention maps are the only output of the detector.

As noted above, the deployment of the first ML model may facilitate real-time usage and/or improved predictions with certain hardware constraints. However, there may be scenarios where it may be acceptable to use the second ML model (e.g., if there is no time and/or hardware constraint). In some cases, the model may be selected (e.g., automatically or manually by a user) based on user requirements and/or conditions. The selection of the first ML model may occur if a small model needs to deployed, less accuracy is needed, a faster speed is needed and/or if the output of the first ML model is to be used in real-time. The selection of the second ML model may occur if a larger model needs to be deployed for higher accuracy and speed/no real-time usage is acceptable.

The following is a detailed description of a possible implementation of the architecture 300 according to certain embodiments.

The following section refers to 'attention distillation' according to an embodiment.

As referred to above, the use of (self)-attention maps may provide a convenient solution for model compression by providing a way to distill large detectors such as DETR into small, fast, and lightweight detectors.

Certain knowledge distillation formulations may allow smaller 'student' models to generalize by taking advantage of 'soft target' class probabilities supplied by a large 'teacher' models. Soft targets from teacher models have higher entropy and can provide more information to student models, as compared to the procedure of training the smaller models on 'hard' ground truth targets.

Self-attention maps extracted from a teacher detection transformer may allow a corresponding learning mechanism for the use-case of distilling object detectors, i.e. they may offer soft probability 'heat maps' that can be used for distillation, in addition to 'hard' bound box labels. By distilling large teacher networks comprising several encoder and decoders layers to smaller single encoder/decoder detection transformers, it may be possible to increase the number of frames per second processed by the student network while only taking a small performance hit compared to using a large teacher network that would otherwise not be suitable for real-time deployment on certain hardware such as ultrasound imaging apparatus.

The following section refers to an 'attention-based detector model' according to an embodiment.

Certain embodiments leverage certain products/output of the DETR architecture. The DETR architecture comprises a backbone convolutional neural network (e.g. ResNet50) that down-samples an input image to produce a tensor of activations that are then processed by an encoder-decoder transformer architecture that directly predicts a set of bounding boxes. Each layer of the transformer encoder-decoder produces an intermediate 'attention map', which is the key component that allows the attention distillation method to work.

The DETR architecture may avoid the need for anchor boxes or non-maximum suppression. Instead, the architecture relies on bipartite matching and imposes a parameter, N, that limits the maximum number of objects that can be detected in an image. For the example purposes described herein, the bipartite matching is trivial, as there is either no object (Ø) or at most only one needle object to detect within an ultrasound frame. Hence, for needle detection embodiments, the limit may be N=1.

The following section refers to '2D-to-2D distillation for images' according to an embodiment.

Fig. 3 described above shows a visual schematic of the attention distillation procedure according to certain embodiments. The dashed arrows 324 show various points in the architecture where knowledge distillation from teacher to student networks 304, 302 may take place. The number of encoder and decoder layers of the student network 302 is smaller and more lightweight than the larger teacher network 304. Both the student and teacher encoder and decoder components 308, 310, 314, 316 produce attention maps. Attention distillation may take place by matching distributions (e.g., 'location information') between the teacher and student attention maps 320, 322. A loss function may be introduced that penalizes student networks when their attention maps do not closely resemble the teacher networks. Distillation can also take place by matching predictions, $\hat{p}$, between teacher and student networks.

The following section refers to '3D-to-2D distillation for videos' according to an embodiment.

Attention distillation can also be used to distill a 3D detector, designed to process a temporal sequence of multiple frames, into a 2D student model that processes only a single frame. 3D detectors may allow temporal information from a sequence of k-input frames to inform bounding box predictions. However, the additional size and complexity of the 3D models, and their reliance on 3D convolution operations, may lead to increased processing times compared to 2D counterparts.

3D-to-2D distillation may allow a 2D student model to ingest temporal information from a 3D teacher, while maintaining low computational complexity.

A possible implementation of a temporal 3D model is to prepend an initial 3-dimensional spatiotemporal convolutional block to the head of an existing object detector. 3D convolution (i.e. 2 spatial and 1 temporal) may be applied repeatably until only a single temporal dimension remains. Other ways to convolve out the temporal dimension, for instance simultaneous temporal and spatial convolution and downsampling, are possible as well. Regardless of the specific backbone design, once a single temporal dimension remains, a 2D object detector may then be applied to predict bounding boxes or other information such as coordinates at detected objects. In some embodiments, the 2D detector head may comprise the attention-based DETR architecture.

The following section refers to 'visual display of distilled attention maps to facilitate human interpretation' according to an embodiment

There may be clinical value in displaying the distilled attention maps generated by the student model, for example to support human visual clinical interpretation. The attention maps may provide a high-content visual representation of salient features, provide transparency into 'black-box' AI-based models and/or provide a mechanism for clinical review. However, it shall be appreciated that a visual display of distilled attention maps (or other types of 'location information') may facilitate human interpretation with other use cases e.g., monitoring industrial processes, etc.

The attention maps (or other types of 'location information') could even be the primary output to be displayed, e.g., replacing the bounding box detection output altogether.

The following section refers to 'selecting or switching between teacher and student models' according to an embodiment

For some use cases, both the larger teacher model and the small student model could be integrated as a deployed model. One or the other model can then be selected depending on whether an immediate real-time result is needed (such as during live ultrasound scanning) or if a small delay can be permitted (such as during review of saved ultrasound loops).

The following section refers to a 'bounding box loss' model according to an embodiment.

For a single instance, y, ground truth class labels and bounding box information is denoted by $y_i = (c_i, b_i)$, where $c_i$ is either Ø or the target class label, e.g., a 'needle', *and* $b_i \in [0,1]$ is a vector that defines the standardized *center_x, center_y, width* and *height* for the ground truth bounding box. The probability of predicting class $c_i \in \{\emptyset, 1\}$, where 1 is the needle class, is given by $\hat{p}_{\psi(i)}(c_i)$ and $\hat{b}_{\psi(i)}$ is the predicted bound box. The bounding box loss is a linear combination of $L_1$ loss and the scale-invariant generalized Intersection-over-Union (IoU) loss $L_{IOU}$. This is shown in Eq. (1) below:

$$L_{box}(b, \tilde{b}) = \lambda_{IOU} L_{IOU}(b, \tilde{b}) + \lambda_{L1}(b - \tilde{b}) \qquad (1)$$

where $\lambda_{iou}, \lambda_{L1}$ are hyper-parameters that control mixing between the loss terms. Eq.(1) combines the two losses, as $L_1$ alone results in different scales for small and large boxes.

The following section refers to a 'loss for attention distillation' model according to an embodiment.

Certain embodiments described herein apply attention distillation by making use of attention matrices generated within the encoder-decoder detection transformer architecture of DETR. A backbone convolutional neural network (e.g. ResNet50) may process an input image and learn a down-sampled feature representation, $f \in R^{C \times H \times W}$. The number of channels in the learned representation is first reduced using $1 \times 1$ convolution and then the $H$ and $W$ dimensions flattened to give the sequence $(x_1, ..., x_n)$, where $x_i \in R^{HW}$ is fed to the detection transformer encoder, along with positional encodings.

Multi-headed scaled dot-product attention is applied to learned query and key matrices ($Q$ and $K$, respectively) by multiplying each $x_i$ in the sequence by network weight matrices, $W^Q$ and $W^K$.

$$A = \text{softmax}\left(\frac{QK^T}{\sqrt{d_k}}\right) \qquad (2)$$

In Eq. (2), $A$ is the attention matrix and $dk$ is the size of multi-headed attention hidden dimension chosen as a hyper-parameter. Certain embodiments described herein select the encoder attention matrix from the final layer of the encoder stack, $A_{enc} \in R^{HW \times HW}$ and the decoder attention matrix from the final layer of the decoder stack, $A_{dec} \in R^{HW}$.

The idea behind attention distillation is to force the encoder or decoder attention matrix of a small student network, $A_s$, to be similar to that of a larger teacher network $A_t$. Attention distillation may use the Kullback Leibler (KL) divergence score between student and teacher attention matrices to accomplish this, as illustrated in Eq. (3).

$$L_{distillation} = (1 - \alpha) \cdot L_{box}(b, \hat{b}) + \alpha \cdot \left( \mathcal{KL}(A_s \| A_t) + T^2 \cdot \mathcal{KL}\left( \sigma\left(\frac{\hat{p}_s}{T}\right) \| \sigma\left(\frac{\hat{p}_t}{T}\right) \right) \right) \quad (3)$$

In Eq. (3), $\alpha$ is a hyper-parameter that controls mixing between the bounding box loss, $L_{box}$ and the attention distillation loss. The first component of the attention distillation loss, $KL(A_s \| A_t)$, applies knowledge distillation to the attention maps created by teacher and student detection transformers. It attempts to match the distribution of the attention maps between teacher and student networks. The attention maps can come from either the encoder, $A_{enc}$, and/or decoder, $A_{dec}$. The second component of the attention distillation loss optionally applies knowledge distillation to the class label predictions,

$$T^2 \cdot \mathcal{KL}\left( \sigma\left(\frac{\hat{p}_s}{T}\right) \| \sigma\left(\frac{\hat{p}_t}{T}\right) \right),$$

where $T$ is a temperature hyper-parameter.

The following section provides evidence of the feasibility of various embodiments described herein. This is done by demonstrating feasibility and efficacy for real-time ultrasound video-based needle detection.

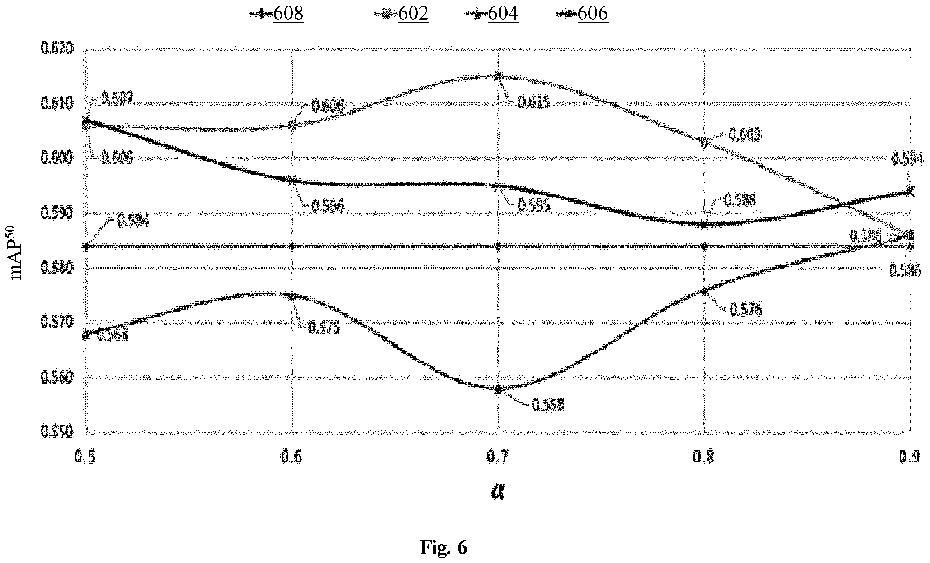

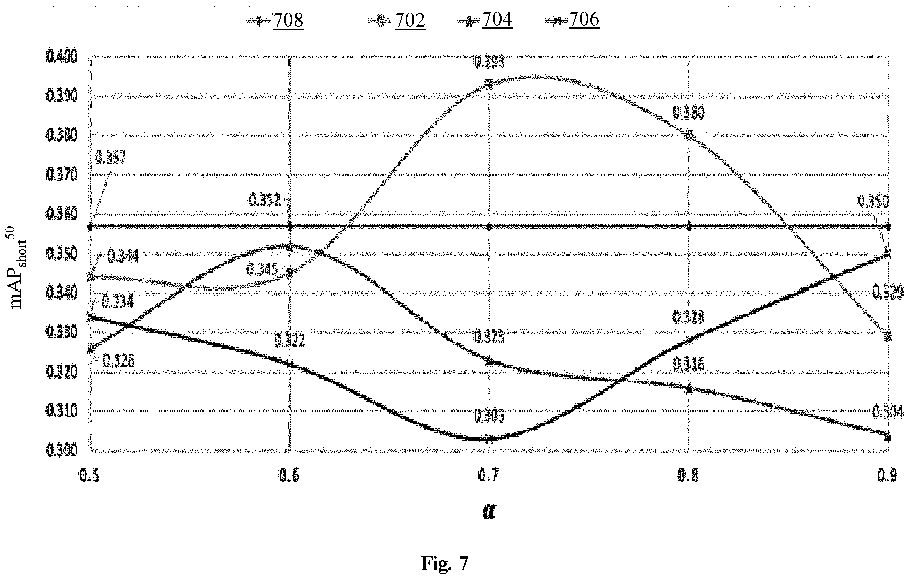

Fig.s 6 and 7 are graphs of data from ultrasound-based needle detection experiments for a series of attention distilled student models where the $\alpha$ parameter varies between values 0.5 - 0.9 (x-axis) with the corresponding average precision results (i.e., 'mAP$^{50}$' in Fig. 6 and 'mAP$_{short}^{50}$' in Fig. 7) for each of the depicted $\alpha$ parameter values when implementing the 'DETR-R50-$n/n$' architecture, described below. The results in Fig. 7 refer to the particularly challenging use-case of 'short' needle insertions, where the needle tip is just barely entering the ultrasound field of view. As can be seen by comparing Figs. 6 and 7, the average precision for each data point is lower in Fig. 7 than the corresponding data points in Fig. 6 in the short needle insertion use-case. Four curves are plotted in each graph, depicting results for 'encoder-only' attention distillation 602, 702 (where the attention map from the final encoder layer is used to train the student network), 'decoder-only' distillation 604, 704 (where the attention map from the final decoder layer is used to train the student network), 'encoder plus decoder' attention distillation 606, 706 (where the final attention distillation loss is calculated as a combination of the loss from the encoder attention map and the loss from the decoder attention map), and a final flat baseline 608, 708 curve when a=0 (which represents no contribution from the attention distillation procedure). These results show, for example, that encoder-only attention distillation results in improved scores over a baseline model that does not use attention distillation. In each case $\alpha$=0.7 gives best performance.

Depending on the mixing (i.e., selected $\alpha$ parameter value) between the bounding box loss, $L_{box}$ and the attention distillation loss, it may be possible to optimize the average precision and other results of the learning process. For example, $\alpha$, may be selected from a range such as between 0.5 and 0.9 as shown by the results in Fig. 6 (for the less challenging use-case). A narrower range, $\alpha$, may be selected from a range such as between 0.6 and 0.9 (e.g., 0.63 and 0.85) as shown by the results in Fig. 7. These values are exemplary and it shall be appreciated that other ranges are possible. In addition, the attention maps produced by the encoder layers may produce improved results especially in the case of certain challenging use cases (e.g., short needle insertion). Thus, in some embodiments, the layer from which the attention map (or other location information) is derived may be selected from: the encoder layer, decoder layer and/or a combination of the encoder plus decoder layer based on whether or not the use case is challenging or associated with less precise results than the baseline.

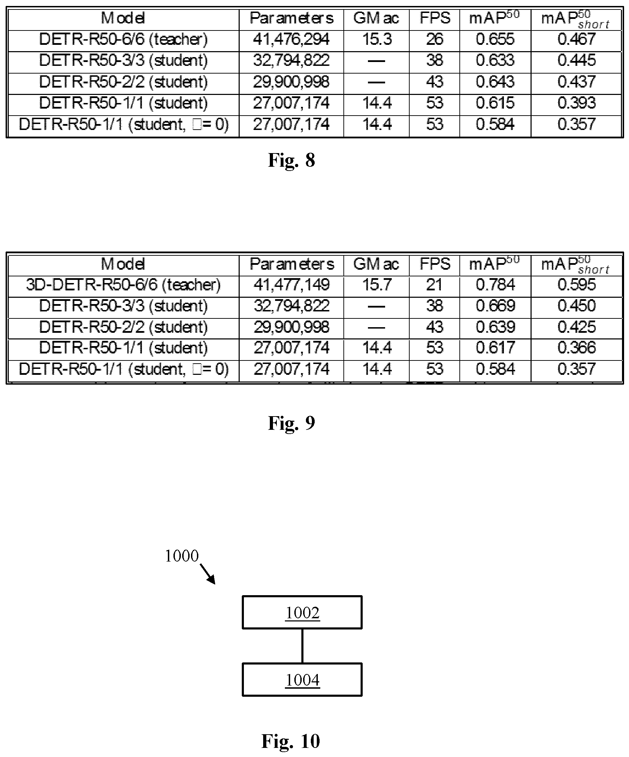

Figs. 8 and 9 are tables highlight the number of parameters in the model (left-hand column), speed results (see columns GMac (giga-multiply-accumulate operations, i.e., the number of multiplication and addition operations undertaken (x 10^9)) and FPS (frames-per-second)) and average precision results for a series of encoder-only student models compared to the performance of the large 2D teacher model (see Table 1), and a large 3D teacher model that incorporates temporal information (see Table 2). Here student models were trained starting with single (1/1) encoder-decoder pairs up to 3/3 (i.e., 1/1 represents a 1 layer encoder paired with a 1 layer decoder; 3/3 represents a 3 layer encoder paired with a 3 layer decoder, etc.). For each student model attention distillation is applied with a rate of $\alpha$=0.7. As the number of encoder-decoder pairs are increased, student models approach the performance of the full teacher model while improving upon the frames per second rate that can be achieved.

Fig. 8 highlights the speed and average precision results of encoder attention distilled student models compared to a large 2D teacher model that incorporate temporal information. DETR-R50-$n/n$ refers to the model type where $n/n$

indicates the number of encoder and decoder layers used in the DETR architecture. The results in Fig. 8 demonstrate that the student models approach the mAP$^{50}$ and mAP$^{50}$ short performance of the full teacher model while improving upon the frames per second rate that can be achieved. For example, the DETR-R50-2/2 student model achieves a mAP$^{50}$ score of 0.643 vs. 0.655, compared to the full teacher model, while improving the FPS from 26 to 43 (e.g., providing the potential for real-time imaging).

Fig. 9 highlights speed and average precision results of encoder attention distilled student models compared to a large 3D teacher model that incorporate temporal information. Figs. 8 and 9 demonstrate some of the performance gains (e.g., in terms of increased speed of the student model compared with the teacher model, as well as potentially comparable accuracy) possible using techniques according to various embodiments described herein.

Embodiments relating to the method 100 and other embodiments for implementing the method 100 are described below.

In some embodiments, the received input data comprises imaging data. In some embodiments, the at least one feature of interest comprises at least one object in the imaging data.

In some embodiments, the first and second ML models are based on a detection transformer architecture. In some embodiments, the at least one layer of the first and second ML models comprises a transformer layer.

In some embodiments, the detection transformer architecture comprises a backbone neural network configured to down-sample the input data to produce a tensor of activations for processing by the at least one transformer layer of the first and second ML models. The at least one transformer layer of the first and second ML models may be based on an encoder-decoder transformer architecture for predicting the location of the at least one feature of interest and/or outputting data representative of the predicted location of the at least one feature of interest.

Fig. 10 refers to a method 1000 of determining a similarity metric according to an embodiment. The method 1000 may be implemented by the same computing device as described in relation to the method 100 (e.g., as part of or in conjunction with the method 100 and/or any other embodiment).

The method 1000 comprises comparing, at block 1002, attention maps generated by the first and second ML models to determine whether or not the first ML model meets a similarity metric indicative of similarity between the compared attention maps. In response to determining that the first ML model does not meet the similarity metric, the method 1000 comprises updating, at block 1004, the at least one layer of first ML model using the at least one attention map generated by the second ML model.

In some embodiments, the similarity metric is based on a Kullback-Leibler, KL, divergence score. However, other similarity metrics may be used, such as L1 or L2 loss.

In some embodiments, the KL divergence score comprises a first component and a second component. The first component may be configured to apply knowledge distillation to the at least one attention map generated by the at least one layer of the first and second ML models by attempting to match the attention maps generated by the first and second ML models. The second component may be configured to apply knowledge distillation to class label predictions.

In some embodiments, the first ML model is updated by modifying a loss function used to train the first ML model based on the similarity metric.

In some embodiments, the loss function is further based on ground-truth target data.

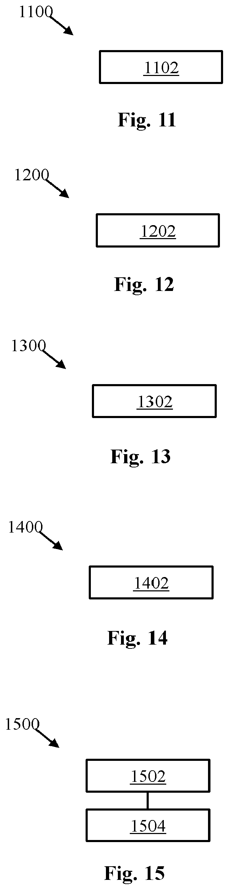

Fig. 11 refers to a method 1100 of using a hyper-parameter according to an embodiment. The method 1100 may be implemented by the same computing device as described in relation to the method 100 (e.g., as part of or in conjunction with the method 100 and/or any other embodiment).

The method 1100 comprises using, at block 1102, a hyper-parameter to control mixing between loss based on the similarity metric and loss based on the ground-truth target labels when training the first and second ML models.

In some embodiments, the at least one attention map generated by the second ML model used to train the first ML model is distilled from the plurality of attention maps generated by the second ML model.

In some embodiments, the at least one attention map generated by the second ML model used to train the first ML model is generated by a final layer of the second ML model.

Fig. 12 refers to a method 1200 of generating an attention map according to an embodiment. The method 1200 may be implemented by the same computing device as described in relation to the method 100 (e.g., as part of or in conjunction with the method 100 and/or any other embodiment).

The method 1200 comprises generating, at block 1202, an attention map representative of the generated location data. The method 1200 uses the first ML model.

In some embodiments, the attention map is generated: by at least one encoder of the at least one layer; by at least one decoder of the at least one layer; or based on a combination of the at least one encoder and decoder of the at least one layer.

Fig. 13 refers to a method 1300 of showing an attention map according to an embodiment. The method 1300 may be implemented by the same computing device as described in relation to the method 100 (e.g., as part of or in conjunction with the method 100 and/or any other embodiment).

The method 1300 comprises causing, at block 1302, a display (e.g., display 210) to show the generated attention map.

In some embodiments, the received input data comprises three-dimensional data and/or temporal data used by the second ML model.

Fig. 14 refers to a method 1400 of implementing a convolution procedure according to an embodiment. The method 1400 may be implemented by the same computing device as described in relation to the method 100 (e.g., as part of or in conjunction with the method 100 and/or any other embodiment).

The method 1400 comprises implementing, at block 1402, a convolution procedure to reduce the received input data to a dimensional format for use by the first ML model.

In some embodiments, the first ML model is trained using input data that has a lower dimensionality than the input data used to train the second ML model.

In some embodiments, training data used to train the first and second ML models is derived from the received input data, previously-used input data and/or historical data.

Fig. 15 refers to a method 1500 of selecting an ML model according to an embodiment. The method 1500 may be implemented by the same computing device as described in relation to the method 100 (e.g., as part of or in conjunction with the method 100 and/or any other embodiment).

The method 1500 comprises receiving, at block 1502, an indication to use the second ML model instead of the first ML model to generate the location data from the received input data. In response to receiving the indication, the method 1500 further comprises generating, at block 1504, the location data using the second ML model.

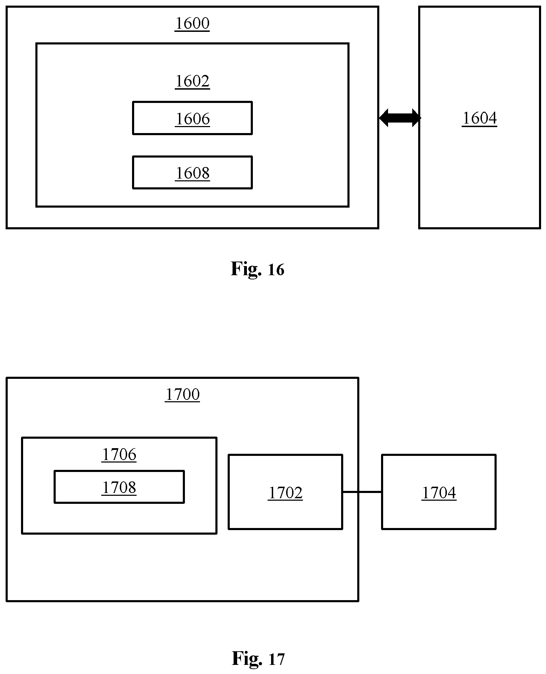

Fig. 16 shows a non-transitory machine-readable medium 1600 for generating location data according to an embodiment. The non-transitory machine-readable medium 1600 comprises instructions 1602 which, when executed on/by at least one processor 1604, cause the at least one processor 1604 to implement certain methods described herein (e.g., methods 100, 1000, 1100, 1200, 1300, 1400, 1500 and/or any other methods described herein). In this embodiment, the instructions 1602 are configured to implement the method 100. The non-transitory machine-readable medium 1600 may be provided in the controller 208 and/or service provider 212 of Fig. 2. In addition, the at least one processor 1604 may be provided in the controller 208 and/or service provider 212 of Fig. 2. Thus, the system 200 may be used for implementing the instructions 1602 comprising the instructions described below.

The instructions 1602 comprise instructions 1606 to receive input data.

The instructions 1602 further comprise instructions 1608 generate location data indicative of a location of any detected at least one feature of interest in the received input data.

The location data is generated using a first machine learning, ML, model configured to detect whether or not there is at least one feature of interest in the received input data.

The first ML model is trained based on a learning process implemented by a second ML model configured to detect whether or not there is at least one feature of interest in the received input data.

The first ML model and the second ML model are each configured to use an attention mechanism to generate; at least one attention map from at least one layer of the first ML model; and a plurality of attention maps from a plurality of layers of the second ML model.

The first ML model comprises fewer layers than the second ML model.

At least one attention map generated by the second ML model is used to train the first ML model.

In some embodiments, the instructions 1602 comprise further instructions to implement any of the other methods described herein.

Fig. 17 shows apparatus 1700 for generating location data according to an embodiment. The apparatus 1700 comprises at least one processor 1702 (e.g., implemented by a computing device such as in the controller 208 and/or service provider 212 depicted by Figure 2). The at least one processor 1702 is communicatively coupled to an interface 1704 for communicating data (e.g., with the radiographic imaging apparatus 202 and/or any other entity with which the at least one processor 1702 may exchange data with during use). In this embodiment, the interface 1704 is configured to receive input data (e.g., from the radiographic imaging apparatus 202) and/or any distilled information. The interface 1704 may be part of the controller 208 and/or service provider 212 referred to in Fig. 2.

The apparatus 1700 further comprises a non-transitory machine-readable medium 1706 storing instructions 1708 readable and executable by the at least one processor 1702 to perform a method corresponding to certain methods described herein (e.g., any of the methods 100, 1000, 1100, 1200, 1300, 1400, 1500 and/or any other methods described herein).

The instructions 1708 are configured to cause the at least one processor 1702 to generate location data indicative of a location of any detected at least one feature of interest in the received input data.

The location data is generated using a first machine learning, ML, model configured to detect whether or not there is at least one feature of interest in the received input data.

The first ML model is trained based on a learning process implemented by a second ML model configured to detect whether or not there is at least one feature of interest in the received input data.

The first ML model and the second ML model are each configured to use an attention mechanism to generate:

at least one attention map from at least one layer of the first ML model; and a plurality of attention maps from a plurality of layers of the second ML model.

The first ML model comprises fewer layers than the second ML model.

At least one attention map generated by the second ML model is used to train the first ML model.

In some embodiments, the instructions 1708 may comprise further instructions to implement any of the other methods described herein.

While the invention has been illustrated and described in detail in the drawings and foregoing description, such illustration and description are to be considered illustrative or exemplary and not restrictive; the invention is not limited to the disclosed embodiments.

One or more features described in one embodiment may be combined with or replace features described in another embodiment.

Embodiments in the present disclosure can be provided as methods, systems or as a combination of machine-readable instructions and processing circuitry. Such machine-readable instructions may be included on a non-transitory machine (for example, computer) readable storage medium (including but not limited to disc storage, CD-ROM, optical storage, etc.) having computer readable program codes therein or thereon.

The present disclosure is described with reference to flow charts and block diagrams of the method, devices, and systems according to embodiments of the present disclosure. Although the flow charts described above show a specific order of execution, the order of execution may differ from that which is depicted. Blocks described in relation to one flow chart may be combined with those of another flow chart. It shall be understood that each block in the flow charts and/or block diagrams, as well as combinations of the blocks in the flow charts and/or block diagrams can be realized by machine readable instructions.

The machine-readable instructions may, for example, be executed by a general-purpose computer, a special purpose computer, an embedded processor, or processors of other programmable data processing devices to realize the functions described in the description and diagrams. In particular, a processor or processing circuitry, or a module thereof, may execute the machine-readable instructions. Thus, functional modules of apparatus and other devices described herein may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate array etc. The methods and functional modules may all be performed by a single processor or divided amongst several processors.

Such machine-readable instructions may also be stored in a computer readable storage that can guide the computer or other programmable data processing devices to operate in a specific mode.

Such machine-readable instructions may also be loaded onto a computer or other programmable data processing devices, so that the computer or other programmable data processing devices perform a series of operations to produce computer-implemented processing, thus the instructions executed on the computer or other programmable devices realize functions specified by block(s) in the flow charts and/or in the block diagrams.

Further, the teachings herein may be implemented in the form of a computer program product, the computer program product being stored in a storage medium and comprising a plurality of instructions for making a computer device implement the methods recited in the embodiments of the present disclosure.

Elements or steps described in relation to one embodiment may be combined with or replaced by elements or steps described in relation to another embodiment. Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method (100), comprising:

receiving (102) input data; and
generating (104) location data indicative of a location of any detected at least one feature of interest in the received input data, wherein:

the location data is generated using a first machine learning, ML, model configured to detect whether or not there is at least one feature of interest in the received input data;

the first ML model is trained based on a learning process implemented by a second ML model configured to detect whether or not there is at least one feature of interest in the received input data;

the first ML model and the second ML model are each configured to use an attention mechanism to generate:

at least one attention map from at least one layer of the first ML model; and
a plurality of attention maps from a plurality of layers of the second ML model;

the first ML model comprises fewer layers than the second ML model; and
at least one attention map generated by the second ML model is used to train the first ML model.

2. The method of claim 1, wherein the first and second ML models are based on a detection transformer architecture, wherein the at least one layer of the first and second ML models comprises a transformer layer.

3. The method of claim 2, wherein the detection transformer architecture comprises a backbone neural network configured to down-sample the input data to produce a tensor of activations for processing by the at least one transformer layer of the first and second ML models, wherein the at least one transformer layer of the first and second ML models are based on an encoder-decoder transformer architecture for predicting the location of the at least one feature of interest and/or outputting data representative of the predicted location of the at least one feature of interest.

4. The method (1000) of any of claims 1 to 3, comprising:

comparing (1002) attention maps generated by the first and second ML models to determine whether or not the first ML model meets a similarity metric indicative of similarity between the compared attention maps; and
in response to determining that the first ML model does not meet the similarity metric, updating (1004) the at least one layer of first ML model using the at least one attention map generated by the second ML model.

5. The method of claim 4, wherein the similarity metric is based on a Kullback-Leibler, KL, divergence score.

6. The method of claim 5, wherein the KL divergence score comprises a first component and a second component, wherein the first component is configured to apply knowledge distillation to the at least one attention map generated by the at least one layer of the first and second ML models by attempting to match the attention maps generated by the first and second ML models, and wherein the second component is configured to apply knowledge distillation to class label predictions.

7. The method (1100) of any of claims 4 to 6, wherein the first ML model is updated by modifying a loss function used to train the first ML model based on the similarity metric, wherein the loss function is further based on ground-truth target data, the method further comprising using (1102) a hyper-parameter to control mixing between loss based on the similarity metric and loss based on the ground-truth target labels when training the first and second ML models.

8. The method of any of claims 1 to 7, wherein the at least one attention map generated by the second ML model used to train the first ML model is distilled from the plurality of attention maps generated by the second ML model.

9. The method (1200) of any of claims 1 to 8, comprising generating (1202), using the first ML model, an attention map representative of the generated location data.

10. The method of claim 9, wherein the attention map is generated:

by at least one encoder of the at least one layer;
by at least one decoder of the at least one layer; or
based on a combination of the at least one encoder and decoder of the at least one layer.

11. The method (1300) of any of claims 9 to 10, comprising causing (1302) a display to show the generated attention map.

12. The method (1400) of any of claims 1 to 11, wherein the received input data comprises three-dimensional data and/or temporal data used by the second ML model, the method further comprising implementing (1402) a convolution procedure to reduce the received input data to a dimensional format for use by the first ML model.

**13.** The method (1500) of any of claims 1 to 12, comprising:
receiving (1502) an indication to use the second ML model instead of the first ML model to generate the location data from the received input data; and in response to receiving the indication, generating (1504) the location data using the second ML model.

**14.** A non-transitory machine-readable medium (1600) storing instructions (1602) executable by at least one processor (1604), wherein the instructions are configured to cause the at least one processor to:

receive (102) input data; and
generate (104) location data indicative of a location of any detected at least one feature of interest in the received input data, wherein:

the location data is generated using a first machine learning, ML, model configured to detect whether or not there is at least one feature of interest in the received input data;
the first ML model is trained based on a learning process implemented by a second ML model configured to detect whether or not there is at least one feature of interest in the received input data;
the first ML model and the second ML model are each configured to use an attention mechanism to generate:

at least one attention map from at least one layer of the first ML model; and
a plurality of attention maps from a plurality of layers of the second ML model;
the first ML model comprises fewer layers than the second ML model; and

at least one attention map generated by the second ML model is used to train the first ML model.

**15.** Apparatus (1700) comprising:

at least one processor (1702) communicatively coupled to an interface (1704), wherein the interface is configured to receive input data; and
a non-transitory machine-readable medium (1706) storing instructions (1708) readable and executable by the at least one processor, wherein the instructions are configured to cause the at least one processor to:
generate location data indicative of a location of any detected at least one feature of interest in the received input data, wherein:

the location data is generated using a first machine learning, ML, model configured to detect whether or not there is at least one feature of interest in the received input data;
the first ML model is trained based on a learning process implemented by a second ML model configured to detect whether or not there is at least one feature of interest in the received input data;
the first ML model and the second ML model are each configured to use an attention mechanism to generate:

at least one attention map from at least one layer of the first ML model; and
a plurality of attention maps from a plurality of layers of the second ML model;
the first ML model comprises fewer layers than the second ML model; and

at least one attention map generated by the second ML model is used to train the first ML model.

100

```
┌─────────────────┐
│       102       │
└─────────────────┘
         │
┌─────────────────┐
│       104       │
└─────────────────┘
```

**Fig. 1**

200

```
        ┌─────────────┐
        │     212     │
        └─────────────┘
               │
┌─────────────┐       ┌─────────────┐         ●
│     208     │───────│     210     │       ／█＼
└─────────────┘       └─────────────┘        206
      ↕
┌──────────────────────────────┐
│        ┌─────┐               │
│    ○   │ 202 │               │
│   ／   │ 204 │─────          │
│        └─────┘─────          │
│        ＼___／                │
└──────────────────────────────┘
```

**Fig. 2**

300

304

312 → 314 → 316 → $(\hat{p}_t, b_t)$

318

320   322

324   324   324

306 → 308 → 310 → $(\hat{p}_s, b_s)$

302

**Fig. 3**

(a)

(b)

Fig. 4

(a)

(b)

**Fig. 5**

**Fig. 6**

**Fig. 7**

EP 4 125 000 A1

| Model | Parameters | GMac | FPS | $mAP^{50}$ | $mAP^{50}_{short}$ |
|---|---|---|---|---|---|
| DETR-R50-6/6 (teacher) | 41,476,294 | 15.3 | 26 | 0.655 | 0.467 |
| DETR-R50-3/3 (student) | 32,794,822 | — | 38 | 0.633 | 0.445 |
| DETR-R50-2/2 (student) | 29,900,998 | — | 43 | 0.643 | 0.437 |
| DETR-R50-1/1 (student) | 27,007,174 | 14.4 | 53 | 0.615 | 0.393 |
| DETR-R50-1/1 (student, □= 0) | 27,007,174 | 14.4 | 53 | 0.584 | 0.357 |

**Fig. 8**

| Model | Parameters | GMac | FPS | $mAP^{50}$ | $mAP^{50}_{short}$ |
|---|---|---|---|---|---|
| 3D-DETR-R50-6/6 (teacher) | 41,477,149 | 15.7 | 21 | 0.784 | 0.595 |
| DETR-R50-3/3 (student) | 32,794,822 | — | 38 | 0.669 | 0.450 |
| DETR-R50-2/2 (student) | 29,900,998 | — | 43 | 0.639 | 0.425 |
| DETR-R50-1/1 (student) | 27,007,174 | 14.4 | 53 | 0.617 | 0.366 |
| DETR-R50-1/1 (student, □= 0) | 27,007,174 | 14.4 | 53 | 0.584 | 0.357 |

**Fig. 9**

1000

1002

1004

**Fig. 10**

1100

```
┌─────────────────┐
│      1102       │
└─────────────────┘
```

**Fig. 11**

1200

```
┌─────────────────┐
│      1202       │
└─────────────────┘
```

**Fig. 12**

1300

```
┌─────────────────┐
│      1302       │
└─────────────────┘
```

**Fig. 13**

1400

```
┌─────────────────┐
│      1402       │
└─────────────────┘
```

**Fig. 14**

1500

```
┌─────────────────┐
│      1502       │
├─────────────────┤
│      1504       │
└─────────────────┘
```

**Fig. 15**

1600

1602

1606

1608

1604

**Fig. 16**

1700

1706

1708

1702

1704

**Fig. 17**

Europäisches
Patentamt

European
Patent Office

Office européen
des brevets

# EUROPEAN SEARCH REPORT

**Application Number**

EP 21 19 6668

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YINGCHAO FENG ET AL: "Double Similarity Distillation for Semantic Image Segmentation", ARXIV.ORG, CORNELL UNIVERSITY LIBRARY, 201 OLIN LIBRARY CORNELL UNIVERSITY ITHACA, NY 14853, 19 July 2021 (2021-07-19), XP091013241, DOI: 10.1109/TIP.2021.3083113 * figures 3, 5 * * page 5, left-hand column * * equation 14 on page 7 * ----- | 1-15 | INV. G06N3/04 G06N3/08 G16H30/40 |
| X | SINA AKBARIAN ET AL: "Evaluating Knowledge Transfer in Neural Network for Medical Images", ARXIV.ORG, CORNELL UNIVERSITY LIBRARY, 201 OLIN LIBRARY CORNELL UNIVERSITY ITHACA, NY 14853, 17 September 2020 (2020-09-17), XP081765711, * figure 2 * ----- | 1,14,15 | |
| A | Carion Nicolas ET AL: "End-to-End Object Detection with Transformers", , 28 May 2020 (2020-05-28), pages 1-26, XP055896603, arXiv.org Retrieved from the Internet: URL:https://arxiv.org/pdf/2005.12872.pdf [retrieved on 2022-03-01] * figure 2 * ----- -/-- | 2,3,10 | TECHNICAL FIELDS SEARCHED (IPC) G06N G16H |

The present search report has been drawn up for all claims

| Place of search | Date of completion of the search | Examiner |
|---|---|---|
| The Hague | 1 March 2022 | Theissing, Simon |

EPO FORM 1503 03.82 (P04C01)

**page 1 of 2**

Europäisches
Patentamt

European
Patent Office

Office européen
des brevets

# EUROPEAN SEARCH REPORT

Application Number

EP 21 19 6668

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHIQING SUN ET AL: "Rethinking Transformer-based Set Prediction for Object Detection", ARXIV.ORG, CORNELL UNIVERSITY LIBRARY, 201 OLIN LIBRARY CORNELL UNIVERSITY ITHACA, NY 14853, 21 November 2020 (2020-11-21), XP081820151, * section 3.1 * | 2,3,10 | |
| A | Wang Wenhui ET AL: "MiniLM: Deep Self-Attention Distillation for Task-Agnostic Compression of Pre-Trained Transformers", , 6 April 2020 (2020-04-06), pages 1-15, XP055896618, arXiv.org Retrieved from the Internet: URL:https://arxiv.org/pdf/2002.10957.pdf [retrieved on 2022-03-01] * figure 1 * | 5,6 | |

TECHNICAL FIELDS
SEARCHED (IPC)

The present search report has been drawn up for all claims

| Place of search | Date of completion of the search | Examiner |
|---|---|---|
| The Hague | 1 March 2022 | Theissing, Simon |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 2 of 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- End-to-end object detection with transformers. **CARION et al.** European Conference on Computer Vision. Springer, 2020, 213-229